# EUROPEAN PATENT SPECIFICATION

(11) **EP 0 319 333 B1**
(45) Date of publication and mention of the grant of the patent: **20.07.1994**
(21) Application number: 88311471.2
(22) Date of filing: 02.12.1988
(51) Int. Cl.: A61K 39/395

(54) **Method for the prevention of graft versus host disease**
Verfahren zur Verhütung von GVHD
Procédé pour la prévention de réaction de rejet

(30) Priority: 02.12.1987 US 127736
(43) Date of publication of application: 07.06.1989
(73) Proprietor: Becton, Dickinson and Company, Franklin Lakes, New Jersey 07417-1880 (US)
(72) Inventor: Reichert, Thomas A., Los Altos, CA (US); Champlin, Richard, Los Angeles, CA (US)
(74) Representative: Ruffles, Graham Keith

(56) References cited:
- EP-A- 0 100 904
- EP-A- 0 240 344
- BIOLOGICAL ABSTRACTS, vol. 84, no. 10, 1987, Biological Abstracts Inc., Philadelphia, PA (US); M.A. COOLEY et al., no. 100147#
- THE LANCET, vol. 25, 1987; pp. 175-178#

## Description

### Field of the Invention

This invention relates to the prevention of or prophylaxis against graft versus host disease (GVHD), and more particularly, to the use of an anti-CD8 monoclonal antibody (MAb) together with Cyclosporine A to prevent GVHD.

### Background of the Invention

GVHD is a sometimes fatal, often debilitating complication that arises in patients who have received allogenic bone marrow transplants. Marrow transplants become necessary in the treatment of certain diseases, such as leukemia, aplastic anemia or certain genetic disorders, in which the patient's own marrow is severly flawed and where total body irradiation or chemotherapy destroy the patient's hematopoietic system. Absent reconstitution of the hematopoietic system, the patient will be severely immunodepressed and susceptible to infection. Hence GVHD is frequently encountered in bone marrow transplantation and presents a major obstacle to the successful treatment of the above disorders.

Working with the H2 histocompatibility system of mice, Korngold and Sprent, Immunological Rev., 71:5 (1983), have reviewed the suspected etiology and pathology of GVHD. Briefly, in its acute form, GVHD is an extraordinarily morbid and often fatal disorder which is primarily, if not exclusively, mediated by T lymphocytes. It typically results from the incomplete immunologic matching of donor with recipient Human Leukocyte antigens (HLA). There are four major HLA antigens: the Class I HLA-A, HLA-B and HLA-C antigens; and the Class II HLA-D region antigens. These antigens form the major histocompatibility complex (MHC), and are expressed in virtually all cells, including nucleated cells in the bone marrow. MHC antigens are cell surface glycoprotens expressed on the lipid membrane. These HLA antigens can trigger the immune system (principally T cells) to respond to foreign antigens. For a more detailed description of the HLA system, see P. Weisz-Carrington, Principles of Clinical Immunohematology, p. 218, YearBook Medical Publishers, Inc. (1986).

Even in those cases where the most complete HLA matching is correctly done, GVHD frequently results. It has been suggested that GVHD results, in those instance, from alloaggression due to minor histocompatibility antigen differences for which many authors have suggested the depletion of donor T cells as a means to avoid GVHD.

Korngold and Sprent were the first to suggest that not all T cells, however, are necessarily involved in inducing GVHD. T cells (CD3⁺) have two major subset populations: T helper/inducer cells (CD4⁺) and T cytotoxic/suppressor cells (CD8⁺). These authors suggested in 1983, on the basis of experiments with a single MHC compatible pair of mouse strains, that the CD8⁺ subset appears to be involved in GVHD. Later, however, B. Hamilton, J. Immunol., 139:2511 (1987) and Korngold and Sprent (1987) reported on experiments in multiple strains of mice. They showed that in some strains merely by depleting T cytotoxic/suppressor cells (Lyt2⁺ in mice) did not abolish GVHD in all mouse strains, but that depletion of L3T4⁺ cells also was required for some strain combinations.

### Brief Description of the Invention

The present invention is founded on a method to prevent GVHD. The method comprises the following steps: in a patient undergoing allogenic bone marrow transplantation, marrow from an HLA-matched donor is treated with anti-CD8 monoclonal antibody to deplete the donor's marrow of T cytotoxic/suppressor cells; the treated marrow then is transplanted into the patient; and a CD4⁺ cell inactivator, such as Cyclosporine A, is given to the patient.

### Detailed Description of the Invention

Patients with either acute myelogenous leukemia (AML), acute lymphoblastic leukemia (ALL) or chronic granulocytic leukemia (CGL) were considered for bone marrow transplantation as part of their therapy. Patients were selected for treatment if they had an HLA A, B and D matched donor. Patients with other types of diseases and with other HLA pairings also may be considered for treatment.

Prior to transplantation, each patient underwent a conditioning regime. The conditioning regime is designed to destroy all malignant cells in the patient. This regime followed standard procedures and included total body irradiation and treatment with anti-neoplastic agents, such as cyclophosphamide (Bristol-Meyers Laboratories) or cytarabine ("ARA-C", UpJohn) and mitoxantrone (Lederle Laboratories). Each type of disease may have a slightly different conditioning regime and care should must be taken to ensure that the proper regime is followed; however, so long as standard pretreatment procedures are employed, the specific conditioning regime is not critical to the practice of the invention. For a review of leukemia conditioning regimes prior to bone marrow transplantation, see Weisz-Carrington, pp. 264-266.

While the patient was undergoing the conditioning regime, bone marrow from the HLA-matched donor was aspirated from the iliac crests. Approximately 3-5 x 10⁸ nucleated marrow cells/kg donor body weight typically were obtained. Mononuclear cells were obtained by Ficoll-Hypaque (Pharmacia) density dependent centrifugation. Mononuclear cells then were suspended in 10% McCoy's medium (American Scientific Products) with 10% patient serum to a concentration of 2 x 10⁷ cells/ml. To the mixture of suspended cells and patient serum was added an amount of an anti-CD8 monoclonal antibody sufficient to deplete the CD8⁺ in the donor's bone marrow. In this example, 10 ug/ml of MAb was used. The antibody may be selected from the group consisting of Anti-Leu-2b (available from Becton Dickinson Immunocytometry Systems), Anti-Leu-2c (hybridoma deposited with the American Type Culture Collection, Rockville, Maryland as HB 9918), OKT8 (available from Ortho Diagnostics, Inc.) or a plurality of such cytotoxic anti-CD8 antibodies. The preferred cytotoxic anti-CD8 monoclonal antibody is produced by the hybridoma deposited as HB 9918.

After 30 minutes, the monoclonal antibody treated cells were washed and resuspended in newborn rabbit complement (Pel-Freeze Biologicals) at 37°C for 45 minutes. The cells then were washed and resuspended in RPMI 1640 (GIBCO), and were placed in a transfusion bag ready for transplantation.

Treatment with anti-CD8 antibody and complement may be repeated to ensure that the CD8⁺ T cells have been depleted. Depletion of the T cells may be checked by immunophenotyping various T cell subsets pre- and post-depletion. This may be done, for example, by using flow cytometry and fluorescently labeled MAbs.

It will be appreciated by those skilled in the art that the anti-CD8 MAb need not be cytotoxic in order to deplete the donor's bone marrow of CD8⁺ cells. A non-cytotoxic anti-CD8 Mab may be linked to a solid phase immunoabsorbant, such as polystyrene beads. The marrow then is passed over the immunoabsorbant to deplete the CD8⁺ cells. Anti-Leu-2a (available from Becton Dickinson Immunocytometry Systems) is one such antibody. Similarly, it will be appreciated that the treatment of the donor's marrow to deplete the CD8⁺ cells may comprise use of an effective amount of a cytotoxic CD8⁺ agent and immunoabsorbation with a CD8⁺ agent. In all cases, the critical element is to deplete the donor's bone marrow of CD8⁺ cells.

Bone marrow cells prepared as above then were transplanted into a conditioned patient. Cells typically were administered I/V over 60-120 minutes. Patients were premedicated with acetominophen (or similar fever depressing drugs) and steroids (such as hydrocortisone). These medications were given to patients to prevent hypersensitivity reactions.

Following transplantation, patients were kept in protective isolation until the absolute granulocyte returned to acceptable levels.

A CD4⁺ cell inactivator then was given. In this example, Cyclosporine A (Sandoz) was given I/V prior to transplantation and continued daily for the next six months to block the function of CD4⁺ cells. Initial doses may be in the range of 2 to 8 mg/kg/day I/V, 3.0 mg/kg was considered optimal, for the first twenty-one days, although dosages were reduced for patients over 35. Patients were switched to oral doses of the same amount for the next three months. The dose then was titered approximately 5% per week until a level of 100 mg/day was achieved. Other cytotoxic CD4⁺ agents such as anti-CD4 MAbs (e.g., Anti-Leu-3a, Becton Dickinson Immunocytometry Systems) may be substituted for Cyclosporine A; however, in all cases, standard procedures should be followed in monitoring a patient's vital and other functions during treatment.

Monoclonal antibody treated bone marrow was examined for T cell depletion. Fluorescein isothiocyanate (FITC) and phycoerythrin (PE) conjugated antibodies (e.g., Anti-Leu-4 and Anti-Leu-2b, respectively) were used to measure T cell (CD8⁺) depletion using standard two color flow cytometric analysis on a FACStar™ flow cytometer (Becton Dickinson Immunocytometry Systems). CD8⁺ cells were reduced to less than 1% of the population of transplanted cells in 14 patients.

Of these 14 patients, two developed grade 2 (cutenous) acute GVHD and one developed grade 3 (rash and diarrhea) acute GVHD. All three patients recovered within 48 hours upon administration of corticosteroids. No deaths from GVHD have been seen. All patients showed sustained engraftment, at the immunologic reconstruction of the patient's hematopoietic system was notable for its balanced recovery of CD4⁺/CD8⁺ cells.

The clone L55 which produces the monoclonal antibody Anti-Leu-2c has been deposited at the ATCC on 1 December 1988 with the number HB 9918.

## Claims

1. The use of at least one anti-CD8 monoclonal antibody and a CD4⁺ cell inactivator for the preparation of a product for use in bone marrow transplant of an allogenic donor matched to a patient for HLA compatability, the product containing:
at least one anti-CD8 monoclonal antibody for depletion of T cytotoxic/suppressor cells in the donor bone marrow,
and a CD4⁺ cell inactivator for inactivating CD4⁺ cells in the patient after receipt of the donor bone marrow,
as a combined preparation of the anti-CD8 monoclonal antibody and CD4⁺ cell inactivator for separate, sequential use in GVHD treatment.

2. The use of Claim 1 wherein the anti-CD8 monoclonal antibody is cytotoxic.

3. The use of Claim 2 wherein the anti-CD8 monoclonal antibody has a pattern of cellular reactivity which is substantially the same as that of the monoclonal antibody produced by the hybridoma deposited as ATCC HB 9918.

4. The use of Claim 1 wherein the anti-CD8 monoclonal antibody is non-cytotoxic.

5. The use of any preceding claim, wherein the CD4⁺ cell inactivator is Cyclosporine A.

6. The use of Claim 1 wherein the CD4⁺ cell inactivator is an anti-CD4 monoclonal antibody.

7. The use of an anti-CD8 monoclonal antibody for the preparation of a pharmaceutical product for use in a method for prevention of or prophylaxis against GVHD in a patient to undergo a bone marrow transplant, where bone marrow of an allogenic donor has been matched to the patient for HLA compatibility, and wherein said method comprises the steps of treating the bone marrow of the donor with said anti-CD8 monoclonal antibody in an amount sufficient to deplete T cytotoxic/suppressor cells, transplanting the treated bone marrow to the patient, and administering to the patient an effective amount of a CD4⁺ cell inactivator sufficient to inactivate CD4⁺ cells.

8. The use of a CD4⁺ cell inactivator for the preparation of a pharmaceutical product for use in a method for prevention of or prophylaxis against GVHD in a patient to undergo a bone marrow transplant, where bone marrow of an allogenic donor has been matched to the patient for HLA compatibility and wherein said method comprises the steps of treating the bone marrow of the donor with anti-CD8 monoclonal antibody in an amount sufficient to deplete T cytotoxic/suppressor cells, transplanting the treated bone marrow to the patient, and administering to the patient an effective amount of said CD4⁺ cell inactivator sufficient to inactivate CD4⁺ cells.

## Patentansprüche

1. Verwendung von mindestens einem monoklonalen anti-CD8-Antikörper und einem CD4⁺-Zellinaktivator zur Herstellung eines Produkts zur Verwendung in Knochenmarktransplantat eines allogenen, in Bezug auf HLA-Verträglichkeit auf einen Patienten abgestimmten Spenders, wobei das Produkt folgende Bestandteile enthält:
mindestens einen monoklonalen anti-CD8-Antikörper zur Verarmung an T-zytotoxischen/Suppressorzellen im Spender-Knochenmark,
und einen CD4⁺-Zellinaktivator zum Inaktivieren von CD4⁺-Zellen im Patienten nach Empfang des Spender-Knochenmarks,
als ein kombiniertes Präparat des monoklonalen anti-CD8-Antikörpers und des CD4⁺-Zellinaktivators zur getrennten sequentiellen Verwendung bei der GVHD-Behandlung.

2. Verwendung nach Anspruch 1, wobei der monoklonale anti-CD8-Antikörper zytotoxisch ist.

3. Verwendung nach Anspruch 2, wobei der monoklonale anti-CD8-Antikörper ein Muster der zellulären Reaktivität aufweist, das im wesentlichen das gleiche Muster wie beim monoklonalen Antikörper, der durch das als ATCC HB 9918 hinterlegte Hybridom gebildet wird, ist.

4. Verwendung nach Anspruch 1, wobei der monoklonale anti-CD8-Antikörper nicht-zytotoxisch ist.

5. Verwendung nach einem der vorstehenden Ansprüche, wobei es sich beim CD4⁺-Zellinaktivator um Cyclosporin A handelt.

6. Verwendung nach Anspruch 1, wobei es sich beim CD4⁺-Zellinaktivator um monoklonalen anti-CD4 -Antikörper handelt.

7. Verwendung des monoklonalen anti-CD8-Antikörpers zur Herstellung eines pharmazeutischen Produkts zur Verwendung in einem Verfahren zur Verhinderung oder Prophylaxe von GVHD bei einem sich einer Knochenmarktransplantation unterziehenden Patienten, wobei Knochenmark eines allogenen Spenders auf den Patienten in Bezug auf HLA-Verträglichkeit abgestimmt worden ist und wobei das Verfahren folgende Stufen umfaßt:
Behandeln des Knochenmarks des Spenders mit dem anti-CD8-monoklonalen Antikörper in einer Menge, die zur Verarmung an T-zytotoxischen/Suppressor-Zellen ausreicht, und
Transplantieren des behandelten Knochenmarks auf den Patienten und Verabreichen einer wirksamen Menge an CD4⁺-Zellinaktivator, die zur Inaktivierung von CD4⁺-Zellen ausreicht, an den Patienten.

8. Verwendung eines CD4⁺-Zellinaktivators zur Herstellung eines pharmazeutischen Produkts zur Verwendung in einem Verfahren zur Verhinderung oder Prophylaxe von GVHD bei einem sich einer Knochenmarktransplantation unterziehenden Patienten, wobei Knochenmark eines allogenen Spenders auf den Patienten in Bezug auf HLA-Verträglichkeit abgestimmt worden ist und wobei das Verfahren folgende Stufen umfaßt:
Behandeln des Knochenmarks des Spenders mit dem anti-CD8-monoklonalen Antikörper in einer Menge, die zur Verarmung an T-zytotoxischen/Suppressor-Zellen ausreicht, Transplantieren des behandelten Knochenmarks auf den Patienten und
Verabreichen einer wirksamen Menge an CD4⁺-Zellinaktivator, die zur Inaktivierung von CD⁺-Zellen ausreicht, an den Patienten.

## Revendications

1. Utilisation d'au moins un anticorps monoclonal anti-CD8 et d'un inactivateur des cellules CD4⁺ pour la préparation d'un produit pour une utilisation dans un transplant de moelle osseuse d'un donneur allogène assorti pour un patient en ce qui concerne la compatibilité HLA, le produit contenant:
au moins un anticorps monoclonal anti-CD8 pour la déplétion des cellules T cytotoxiques/suppressives dans la moelle osseuse du donneur
et un activateur des cellules CD4⁺ pour inactiver les cellules CD4⁺ chez le patient après réception de la moelle osseuse du donneur
sous la forme d'une préparation associée de l'anticorps monoclonal anti-CD8 et de l'inactivateur des cellules CD4⁺ pour une utilisation séparée et séquentielle dans un traitement du GVHD.

2. Utilisation de la Revendication 1, dans laquelle l'anticorps monoclonal anti-CD8 est cytotoxique.

3. Utilisation de la Revendication 2, dans laquelle l'anticorps monoclonal anti-CD8 a un type d'activité cellulaire qui est substantiellement le même que celui de l'anticorps monoclonal produit par l'hybridome déposé sous la référence ATCC HB 9918.

4. Utilisation de la Revendication 1, dans laquelle l'anticorps monoclonal anti-CD8 est non-cytotoxique.

5. Utilisation de l'une quelconque des revendications précédentes, dans laquelle l'inactivateur des cellules CD4⁺ est la cyclosporine A.

6. Utilisation de la Revendication 1, dans laquelle l'inactivateur des cellules CD4⁺ est un anticorps monoclonal anti-CD4.

7. Utilisation d'un anticorps monoclonal anti-CD8 pour la préparation d'un produit pharmaceutique pour une utilisation dans une méthode de prévention ou de prophylaxie contre le GVHD chez un patient qui va être soumis à un transplant de moelle osseuse, où la moelle osseuse d'un donneur allogène a été assortie pour le patient en ce qui concerne la compatibilité HLA et dans laquelle ladite méthode comprend les étapes de traitement de la moelle osseuse du donneur avec ledit anticorps monoclonal anti-CD8 en une quantité suffisante pour épuiser les cellules T cytotoxiques/suppressives, de transplantation de la moelle osseuse traitée au patient et d'administration au patient d'une quantité efficace d'un inactivateur des cellules CD4⁺ suffisante pour inactiver les cellules CD4⁺.

8. Utilisation d'un inactivateur des cellules CD4⁺ pour la préparation d'un produit pharmaceutique pour une utilisation dans une méthode pour la prévention ou la prophylaxie contre le GVHD chez un patient qui va être soumis à un transplantation de moelle osseuse, où la moelle osseuse d'un donneur allogène a été assortie pour le patient en ce qui concerne la compatibilité HLA et dans laquelle ladite méthode comprend les étapes de traitement de la moelle osseuse du donneur avec un anticorps monoclonal anti-CD8 en une quantité suffisante pour épuiser les cellules T cytotoxiques/suppressives, de transplantation de la moelle osseuse traitée au patient et d'administration au patient d'une quantité efficace dudit inactivateur de cellules CD4⁺ en une quantité suffisante pour inactiver les cellules CD4⁺.
